# EUROPEAN PATENT SPECIFICATION

(11) **EP 2 965 727 B1**
(45) Date of publication and mention of the grant of the patent: **26.12.2018**
(21) Application number: 14176607.1
(22) Date of filing: 10.07.2014
(51) Int. Cl.: A61F 5/058

(54) **Adaptable protective device**
Anpassbare Schutzvorrichtung
Dispositif de protection adaptable

(43) Date of publication of application: 13.01.2016
(73) Proprietor: Advance Kites S.r.l., 25057 Sale Marasino (BS) (IT)
(72) Inventor: Mazzucchelli, Alessandro, 25057 Sale Marasino (Brescia) (IT)
(74) Representative: Lunati & Mazzoni S.r.L.

(56) References cited:
- EP-A1- 1 946 726
- EP-A1- 2 492 211
- US-A1- 2002 077 539
- US-A1- 2004 073 152

## Description

Subject of the present invention is an adaptable protective device of the type specified in the first claim.

In particular, the invention concerns a particular device suitable to protect and immobilize a portion of the human body or an object.

Various types of protective devices are presently known, as plasters and rigid bandages for persons for healing purposes, rigid boots for persons for sport activities, casings for fragile items and the like, and more.

For example various devices exist made of deformable and hardening materials, such as polymers, hardening foams and more. Such materials can adapt to the shape of the item to protect when in a deformable state, and then they stiffen and protect the item contained within.

The protection conferred by these is particularly due to the fact, that such devices distribute any external shocks in an uniform way on the entire element they protect.

Other protective devices are rigid bandages for orthopedic injuries, as well as so called plasters, Tensoplast and similar. Such devices also make use of a deformable item, which is then hardened by a triggered chemical reaction.

The item being deformable, it is possible to adapt the same to the shape of the portion of the body to bandage or immobilize.

Such devices have the drawback of not perfectly protecting and not permitting a reuse, being disposable.

An important development has been carried out by the same Applicant. Such innovation is described in Patents WO-A-2012/114300 and WO-A-2012/114301, in which items are described including inner volumes containing rigid particles. From such volumes air can also be extracted, so that due to the depression the rigid particles become compact by making a rigid counter-shaped body with respect to the shape of the item on which they have been placed.

Similar devices, including a device according to the preamble of claim 1, and even for different purposes, are described in Patent WO-A-2012/114301.

However, the first of such documents describes a protective item capable of only partially wrapping the item it protects. Such solution is optimal in many, but not in all cases. For example, such solution is not optimal for immobilizing limbs and similar, for which it is important to completely wrap the entire limb at 360° and not only a portion of the same. In fact, if one of such devices is wrapped around a cylindrical item with a broadly-angled circumferential sector, for example greater than the flat angle, during the air suction each portion of the protective device contracts approaching its own centre, so moving away from the item to protect and not completely performing the protection.

Furthermore, the second of such documents describes a device which permits to completely protect only items comprised and contained within a casing. The same solution is therefore not applicable to human limbs, portions of elongated items or of great dimension and other more. In this situation the technical task of the present invention is to conceive an adaptable protective device suitable to substantially obviate the cited drawbacks.

Within said technical task an important aim of the invention is to provide an adaptable protective device which precisely adapts to the portion of an item around which it wraps, even if it is wrapped for broad angles.

Another important aim of the invention is to conceive a protective device usable several times.

The technical task and the specified aims are reached by an adaptable protective device as claimed in annexed Claim 1.

Preferred embodiments are highlighted in the sub-claims.

The features and advantages of the invention are explained in the following by the detailed description of a preferred embodiment of the invention, with reference to the annexed drawings, in which:
**Fig. 1a** shows a sagittal section of an adaptable protective device according to the invention in a first configuration;
**Fig. 1b** shows a sagittal section of the adaptable protective device according to the invention in a second configuration;
**Fig. 1c** shows a sagittal section of the adaptable protective device according to the invention in a third configuration;
**Fig. 1d** shows a sagittal section of the adaptable protective device according to the invention in a fourth configuration;

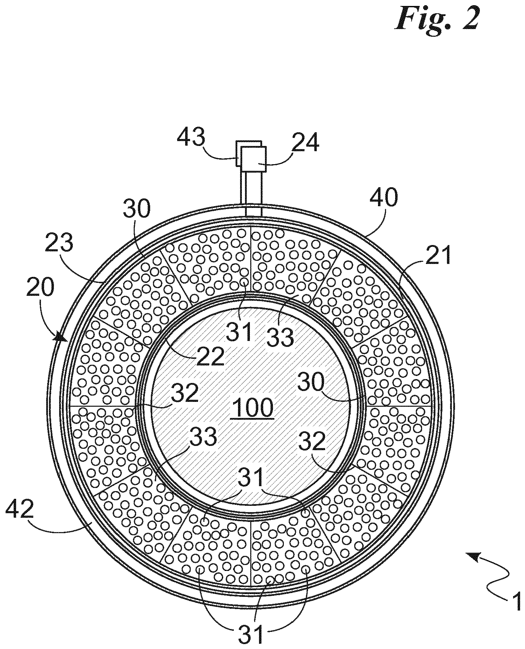

**Fig. 2** is the cross section showing a portion of the adaptable protective device according to the invention, in the configuration of Figure 1a;
**Fig. 3** shows a sagittal section of a variant of the adaptable protective device in the configuration of Fig. 1d.

With reference to cited Figures, the adaptable protective device according to the invention is indicated as a whole with **1.**

It is suitable to include a portion of the body to protect **100,** in particular a portion of a human body, such as a limb, a foot or hand for medical purposes or as a simple support (shoes, various protections) or a portion of a body made of a device which is not a portion of a human and/or animal body, for example of a mechanical or electronic item of any other type and shape. The same device 1 is suitable in all those cases in which the same cannot be entirely contained within a closure casing. The device 1 can then be either a protection of a portion of a human or animal body, or a protection of an object.

The device 1 comprises a main casing **20** impermeable to the passage of fluid, flexible and defining a main volume **21.** It is for example made of a polymeric and elastic membrane, so that the protective device 1 adapts to the shape of the portion of the body with which the same device 1 is associated. In detail, it is preferably made either of a EVA foam with closed cells, polychloroprene or Neoprene®, polyvinylchloride or of composite materials and more.

The main casing 20 is suitable to be placed at least partially around said portion of body to protect 100, so that said portion of body to protect 100 is at the outside of said main volume 21. The main casing 20 also defines an inner surface **22** substantially in contact with said portion of body to protect 100 and an outer surface **23** not in contact with said portion of body to protect 100.

The device 1 also comprises an inner casing **30** placed at the inside of the main casing 20 and permeable to the passage of fluid, and flexible. The inner casing 30 in turn includes a plurality of filler particles **31.** The inner casing 30 preferably has a plurality of intermediate casings **33** mutually separated through walls **32** permeable to the passage of fluid and flexible and preferably made of the same material of the inner casing 30. The inner casing 30 and the walls 32 are preferably completely permeable to fluids, then they are made of permeable materials due to their intrinsic and microscopic features, such as for instance textiles. In particular, they are suitably elastic so to permit, as better described in the following, to define the relaxed and compressed configuration. More in particular, they are elastic textiles such as Lycra® or similar.

The intermediate casings 33 permit to uniformly place the filler particles 31 and avoid that the same accumulate just in a few portions of the inner casing 30. The walls 32 then permit to obtain intermediate casings 33 having selected sizes in the three dimensions, in particular according to the height given by the height of the single walls 32.

The particles 31 can have various shapes, materials and dimensions and be of different types in order to define a particular physical feature of the assembly of particles in a compressed configuration.

In particular one type of particles can be characterized by particles 31 made of the same material and having for example dimensions and shapes also variable from one particle to the other.

Another type of particles 31 can be characterized by particles 31 all having the same shape and dimension, and possibly also made of partially different materials but having similar mechanical characteristics.

A further type can also be made of very homogeneous particles 31 all having certain sizes, shapes and materials, and mutual variations comprised within strict tolerances.

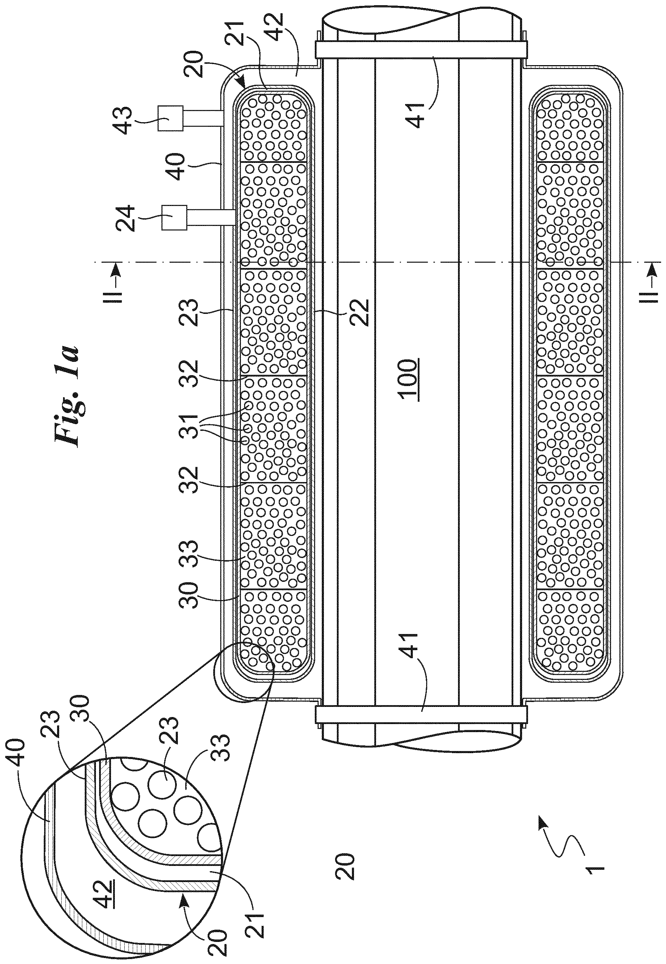

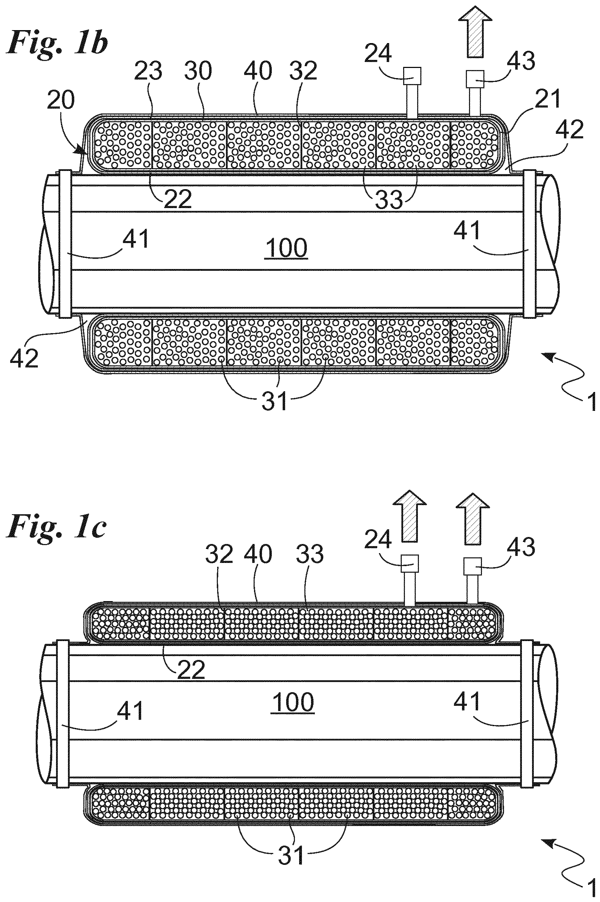

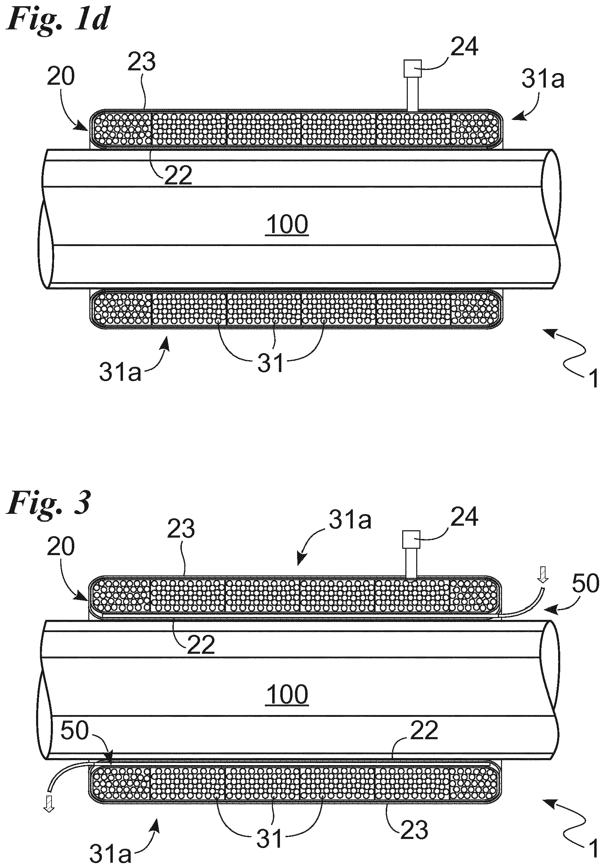

The main casing 20 then comprises a first valve **24** suitable to realize or interrupt a connection for fluid passage, preferably gas and better air, between the main volume 21 and the external environment and suitable to permit the depressurization of said main volume 21, and consequently also of the inner casing 30 and of the various intermediate casings 33, so defining a relaxed configuration (Fig. 1a, Fig. 1b), in which the filler particles 31 are movable inside said inner casing 30; and a compressed configuration (Fig. 1d) in which said filler particles 31 are compacted and substantially define at least one solid body **31a.**

Thanks to said partition of the types of particles, it is possible to obtain solid bodies 31a with different physical and mechanical features according to the type of particles 31. For example in a medical plaster it is possible to place smaller or more elastic particles 31, which make softer and/or more elastic bodies 31a, at the skin or less resistant body parts like fingers, and more rigid or bigger particles 31, which make stiffer and more resistant bodies 31a, at a distance from the skin or at body portions like elbows or similar. The same argument applies to objects having stiff and fragile portions.

The device 1 also comprises a closure casing **40** impermeable to the passage of fluid and flexible and preferably elastic. It can be made of a polymeric membrane, like those for making bags or more.

The closure casing 40 comprises sealing means **41** suitable to create a fluid seal between said closure casing 40 and said portion of body to protect 100, consequently making a closure volume **42,** impermeable to the passage of fluid, and including said main casing 20 and said portion of body to protect 100.

The sealing means 41 can be simple elastic bands, one or two and more depending on the shape of the body and of the portion of body to protect 100. They close the closure casing 40 on the body to protect 100.

The casing 40 also comprises a second valve **43** suitable to realize or interrupt a fluid passage connection, preferably gas and better air, between the closed volume 42 and the external environment and suitable to permit to depressurize said closed volume 42 by defining a closure configuration (Fig. 1c, Fig. 1d) in which the closure casing 40 is depressurized and the atmospheric pressure pushes, in any direction, the inner casing 41 against the portion of body to protect 100. The first valve 24 also comprises connection means with the closure casing 40 suitable to permit the passage of the same through the casing 40 and one of its controls from outside. For example, a particular free solvable connection is possible between the valve and the casing 40 and another valve connected with the casing 40 or other more.

The valves 24 and 43 are finally preferably one-way-valves suitable to permit the inlet and not the outlet of air from the casings 40 and 20, unless a different control, similar to that of the inner tubes for tires but operating in an opposite direction.

The adaptable protective device 1 also comprises a heat exchange channel **50,** partially disposed inside said main casing 20 and in particular interposed between the inner casing 30 and the main casing 20 at the inner surface. It is preferably flexible and made of a polymeric material and is suitable to regulate the temperature of the portion of body to protect 100, thanks to the possibility of flowing a fluid, in particular a liquid, to the inside of the same, at the desired temperatures. The heat exchange channel 50 suitably follows a serpentine path.

The invention also comprises a new method of protection of a portion of a body to protect 100, preferably done with the adaptable protective device 1 described before and coincident with the method of utilization of the same device.

Such method comprises the step of placing the main casing 20, including the inner casing 30 with the various intermediate casings 33 and particles 31, around the portion of body to protect 100.

The closure casing 40 is then placed around the outer surface 23 of the main casing 20, and sealing means 41 are placed suitable to make a fluid sealing between the closure casing 40 and the portion of body 41. The sealing means 41 and the closure casing 40 consequently make the closed volume 42 impermeable to the fluid passage and including the main casing 20, in turn including the inner casing 30 with the various intermediate casings 33 and particles 31, and the portion of body to protect 100. Such configuration is illustrated in Fig. 1a. Furthermore in such configuration the particles 31 are free to move within the intermediate casings 33 and so the inner casing 30 and consequently the inner casing 30 and the main casing 20 and the closure casing 40 are freely adaptable to the shape of the portion of body to protect 100.

Subsequently, as shown in Fig. 1b, the closure volume 42 is depressurized through the first valve 24, by defining a closure configuration in which the closure casing 40 is pushed by the atmospheric pressure and in turn it pushes in any direction, against the main casing 20 and against the inner casing 30 and the particles 31 and consequently pushes the device 1 against the portion of body to protect 100.

Still subsequently, or at the same time with the preceding step, the main volume 21 is depressurized through the second valve 43, as illustrated in Fig. 1c, by defining a compressed configuration in which the filler particles 31 are compacted and substantially define at least one solid body 31a, perfectly profiled regarding the shape of the portion of body to protect 100.

In a last step the valves 24 and 43 can be automatically closed or not, and the closure casing 40 can be moved away from the remainder of the device 1, as illustrated in Fig. 1d.

The invention permits important advantages.

In fact, the device 1 permits to perfectly conform the solid bodies 31a to the shape of a portion of body to protect 100, even if it is not contained within a closed casing.

Such solution works not only in a restricted area, but it is possible to wrap at 360° the portion of body to protect 100.

Such advantage is determined by the uniform and omnidirectional thrust of the atmospheric pressure.

It is then possible to plaster with impeccable precision a portion of a human body and also protect a portion of an object.

Another advantage is due to the fact that it is not necessary to place under vacuum the portion of body 100 if not during the presetting of the device 1.

Another advantage is also due to the heat exchange channel 50 which permits to regulate the temperature of a portion of human body or an object by causing the channel 50 to perfectly abut against the portion of body 100.

The device 1 is finally usable many times, in order to return to the starting configuration (Fig. 1a); in fact, it is sufficient to open the valve 24 permitting the passage of air to the inside of the casing 20.

Due to such advantages, the device 1 can be applied for example: in special packages, such as for transporting organs, unstable liquids (nitroglycerine), weapons, explosives and similar.

Another exemplary embodiment can be a corset for disables, which can stiffen just when necessary.

A further example can be a support for legs for persons having mobility problems.

A further example are armors, for example bulletproof vests, also for women who having breasts risk very serious trauma due to the low ergonomics of the traditional protections, problem obviated through the device 1.

The invention is susceptible of variants all within the inventive concept. All described items can be substituted with equivalent items and details, materials, shapes and dimensions can be of any kind.

## Claims

1. Adaptable protective device (1) suitable to include a portion of body to protect (100) comprising
- a main casing (20) impermeable to the passage of fluid, flexible and defining a main volume (21), said main casing (20) being suitable to be placed at least partially around said portion of body to protect (100) so that said portion of body to protect (100) is outside said main volume (21),
- said main casing (20) defining an inner surface (22) substantially in contact with said portion of body to protect (100) and an outer surface (23) not in contact with said portion of body to protect (100),
- an inner casing (30) placed inside said main casing (20), permeable to the passage of fluid and flexible;
- a plurality of filler particles (31) housed in said inner casing (30);
- a first valve (24) suitable to form or interrupt a connection for fluid passage between said main volume (21) and the external environment and suitable to permit the depressurisation of said main volume (21) defining a relaxed configuration in which said filler particles (31) are movable inside said at least one inner casing (30); and a compressed configuration in which said filler particles (31) are compacted and substantially define at least one solid body,
- **characterised in that** it comprises
- a closure casing (40) impermeable to the passage of fluid, flexible and comprising sealing means (41) suitable to create a fluid seal between said closure casing (40) and said portion of body to protect (100) thereby forming a closed volume (42) impermeable to the passage of fluid and including said main casing (20) and said portion of body to protect (100),
- a second valve (43) suitable to form or interrupt a connection for fluid passage between said closure volume (42) and the external environment and suitable to permit the depressurisation of said closure volume (42) defining a closed configuration in which said closure casing (40) is depressurised and the atmospheric pressure presses, in all directions, said inner casing (41) against said portion of body to protect (100).

2. Adaptable protective device (1) as claimed in claim 1, comprising a heat exchange channel (50), inside said main casing (20) and suitable to adjust the temperature of said portion of body to protect (100).

3. Adaptable protective device (1) as claimed in one or more of the preceding claims in which said inner casing (30) comprises a plurality of intermediate containers (33).

4. Adaptable protective device (1) as claimed in the previous claim, in which said intermediate containers (33) are reciprocally separated by walls (32).

5. Adaptable protective device (1) as claimed in one or more of the preceding claims in which said inner casing (30) is elastic.

6. Adaptable protective device (1) as claimed in one or more of the preceding claims in which said first valve (24) comprises means of connection with the closure casing (40) suitable to enable the passage of said first valve (24) through the closure casing (40) and its control from the outside.

7. Protection method of a portion of body to protect (100) constituted by a device not being a portion of human and/or animal body, enacted by means of an adaptable protective device (1)
said adaptable protective device(1) comprising:
- a main casing (20) impermeable to the passage of fluid , flexible and defining a main volume (21),
- an inner casing (30) placed inside said main casing (20), permeable to the passage of fluid and flexible;
- a plurality of filler particles (31) housed in said inner casing (30);
said method comprising:
- placing said main casing (20), including said inner casing (30), around said portion of body to protect (100), defining an inner surface (22) substantially in contact with said portion of body to protect (100) and an outer surface (23) not in contact with said portion of body to protect (100),
- placing a closure casing (40), impermeable to the passage of fluid and flexible, around said outer surface (23) of said main casing (20),
- placing sealing means (41) suitable to create a fluid seal between said closure casing (40) and said portion of body to protect (41) thereby forming a closure volume (42) impermeable to the passage of fluid and including said main casing (20) and said portion of body to protect (100),
- depressurising said closure volume (42) defining a closed configuration in which said closure casing (40) is depressurised and the atmospheric pressure presses, in all directions, said inner casing (41) against said portion of body to protect (100),
- depressurising said main volume (21), not before depressurising said closure volume (42), defining a compressed configuration in which said filler particles (31) are compacted and substantially define at least one solid body.

8. Protection method of a portion of body to protect (100) as claimed in the previous claim, comprising a final step of distancing said closure casing (40) from the remaining part of the adaptable protective device (1).

## Patentansprüche

1. Anpassbare Schutzvorrichtung (1) zum Einschluss eines Teils des zu schützenden Körpers (100), die Folgendes umfasst:
- einen Hauptmantel (20), der für den Durchgang von Fluid undurchlässig ist, der flexibel ist und ein Hauptvolumen (21) definiert, wobei dieser Hauptmantel (20) dazu geeignet ist, zumindest teilweise um den zu schützenden Abschnitt des Körpers (100) herum platziert zu werden , so dass dieser Abschnitt des zu schützenden Körpers (100) außerhalb des Hauptvolumens (21) liegt,
- wobei der Hauptmantel (20) eine Innenfläche (22) definiert, die im Wesentlichen in Kontakt mit dem Abschnitt des zu schützenden Körpers (100) steht, und eine Außenfläche (23), die nicht in Kontakt mit dem Abschnitt des zu schützenden Körpers (100) steht,
- einen Innenmantel (30), der innerhalb des genannten Hauptmantels (20) angeordnet, für den Durchgang von Fluid durchlässig und flexibel ist;
- eine Vielzahl von Füllteilchen (31), die in diesem Innenmantel (30) untergebracht sind;
- einem ersten Ventil (24), das eine Fluidströmungsverbindung zwischen dem Hauptvolumen (21) und der äußeren Umgebung herstellen oder unterbrechen kann und die Druckentlastung des Hauptvolumens (21) ermöglichen kann, das eine entspannte Konfiguration definiert, in der die Füllteilchen (31) innerhalb mindestens des einen Innenmantels (30) beweglich sind; und eine komprimierte Konfiguration, in der die Füllteilchen (31) verdichtet sind und im Wesentlichen mindestens einen festen Körper darstellen,
- **gekennzeichnet dadurch, dass** sie Folgendes umfasst:
- einen Verschlussmantel (40), der für den Durchgang von Fluid undurchlässig ist, der flexibel ist und Abdichtmittel (41) beinhaltet, die dazu bestimmt sind, eine Fluidabdichtung zwischen dem Verschlussmantel (40) und dem zu schützenden Abschnitt des Körpers (100) zu erreichen, wodurch ein für den Durchgang von Fluid undurchlässiges Schließvolumen (42) erzeugt wird, das den Hauptmantel (20) und den zu schützenden Teil des Körpers (100) einschließt,
- ein zweites Ventil (43), das dazu bestimmt ist, eine Fluidströmungsverbindung zwischen dem Schließvolumen (42) und der äußeren Umgebung herzustellen oder zu unterbrechen, und das dazu geeignet ist, die Druckentlastung des Schließvolumens (42) zu ermöglichen , indem es eine Schließkonfiguration definiert, in welcher der Schließmantel (40) drucklos gemacht wird und der Atmosphärendruck den Innenmantel (41) in jeder Richtung gegen den zu schützenden Teil des Körpers (100) drückt.

2. Anpassbare Schutzvorrichtung (1) nach Anspruch 1, mit einem Wärmeaustauschkanal (50) innerhalb des Hauptmantels (20), der in der Lage ist, die Temperatur des zu schützenden Teils des Körpers (100) zu regeln.

3. Anpassbare Schutzvorrichtung (1) nach einem oder mehreren der vorstehenden Ansprüche, bei welcher der Innenmantel (30) eine Mehrzahl von Zwischenbehältern (33) umfasst.

4. Anpassbare Schutzvorrichtung (1) nach dem vorstehenden Anspruch, bei der diese Zwischenbehälter (33) durch Wände (32) voneinander getrennt sind.

5. Anpassbare Schutzvorrichtung (1) nach einem oder mehreren der vorstehenden Ansprüche , bei der Innenmantel (30) elastisch ist.

6. Anpassbare Schutzvorrichtung (1) nach einem oder mehreren der vorhergehenden Ansprüche, bei der das erste Ventil (24) Verbindungsmittel mit dem Schließmantel (40) zur Ermöglichung des Durchgangs dieses ersten Ventils (24) durch den Schließmantel (40) und dessen Steuerung von außen umfasst.

7. Verfahren zum Schutz eines zu schützenden Teils eines Körpers (100), bestehend aus einer Vorrichtung, die kein Teil des menschlichen und/oder tierischen Körpers ist, und die durch eine anpassbare Schutzvorrichtung (1) bereitgestellt wird,
wobei die anpassbare Schutzvorrichtung (1) Folgendes beinhaltet:
- einen Hauptmantel (20), der für den Durchgang von Fluid undurchlässig ist, flexibel ist und ein Hauptvolumen (21) definiert,
- einen Innenmantel (30), der innerhalb des genannten Hauptmantels (20) angeordnet, für den Durchgang von Fluid durchlässig und flexibel ist;
- eine Vielzahl von Füllteilchen (31), die in diesem Innenmantel (30) untergebracht sind;
wobei dieses Verfahren Folgendes umfasst:
- Anordnung des Hauptmantels (20) einschließlich des Innenmantels (30), um den zu schützenden Teil des Körpers (100) herum , wobei er eine Innenfläche (22) definiert, die im Wesentlichen in Kontakt mit dem Teil des zu schützenden Körpers (100) steht, und eine Außenfläche (23), die nicht in Kontakt mit dem zu schützenden Teil des Körpers (100) in Kontakt steht,
- Bereitstellung eines Verschlussmantels (40), der für den Durchgang von Fluid undurchlässig und flexibel ist, um die Außenfläche (23) dieses Hauptmantels (20) herum,
- Bereitstellung von Abdichtungsmitteln (41), um eine Fluiddichtheit zwischen dem Verschlussmantel (40) und dem zu schützenden Teil des Körpers (41) zu erreichen, wodurch ein für den Durchgang von Fluid undurchlässiges Schließvolumen (42) erzeugt wird, welches den Hauptmantel (20) und den zu schützenden Teil des Körpers (100) einschließt,
- Druckentlastung dieses Schließvolumens (42) durch Definition einer Schließkonfiguration, in welcher der Schließmantel (40) drucklos gemacht wird und der Atmosphärendruck den Innenmantel (41) in jeder Richtung gegen den zu schützenden Teil des Körpers (100) drückt,
- Druckentlastung des Hauptvolumens (21) nicht vor der Druckentlastung des Schließvolumens (42), was eine komprimierte Konfiguration definiert, in der die Füllteilchen (31) verdichtet sind und im Wesentlichen mindestens einen festen Körper definieren.

8. Verfahren zum Schutz eines Teils des zu schützenden Körpers (100) nach dem vorherigen Anspruch, jedoch einschließlich einer Endphase der Entfernung des Schließmantels (40) vom verbleibenden Teil der anpassbaren Schutzvorrichtung (1).

## Revendications

1. Dispositif de protection adaptable (1) pouvant inclure une partie du corps à protéger (100) comprenant
- une enveloppe principale (20) imperméable au passage de fluide, souple et définissant un volume principal (21), appelée enveloppe principale (20) pouvant être placée au moins partiellement autour de la partie du corps à protéger (100) de sorte que la partie du corps à protéger (100) se trouve à l'extérieur du volume principal (21),
- l'enveloppe principale (20) définissant une surface interne (22) sensiblement en contact avec la partie du corps à protéger (100) et une surface extérieure (23) non en contact avec la partie du corps à protéger (100),
- une enveloppe intérieure (30) située à l'intérieur de l'enveloppe principale (20), perméable au passage de fluide et souple ;
- plusieurs particules de remplissage (31) établies dans l'enveloppe intérieure (30);
- une première soupape (24) ayant pour fonction d'établir ou d'interrompre une connexion d'écoulement de fluide entre le volume principal (21) et l'environnement extérieur et pour permettre la dépressurisation du volume principal (21) définissant une configuration détendue, dans laquelle les particules de remplissage (31) sont mobiles au moins dans le volume d'une enveloppe interne (30) ; et une configuration comprimée dans laquelle les particules de remplissage (31) sont compactées et définissent sensiblement au moins un corps solide,
- **caractérisé par**
- une enveloppe de fermeture (40) imperméable au passage de fluide, souple et comportant des moyens d'étanchéité (41) conçus pour réaliser une étanchéité de fluide entre l'enveloppe de fermeture (40) et la partie du corps à protéger (100) créant ainsi un volume de fermeture (42) imperméable au passage du fluide et comportant l'enveloppe principale (20) et la partie du corps à protéger (100),
- une deuxième soupape (43) ayant pour fonction d'établir ou interrompre une connexion d'écoulement de fluide entre le volume de fermeture (42) et l'environnement extérieur et pour permettre la dépressurisation du volume de fermeture (42) en définissant une configuration de fermeture dans laquelle l'enveloppe de fermeture (40) est dépressurisée et la pression atmosphérique presse, dans chaque direction, sur l'enveloppe intérieure (41) contre la partie du corps à protéger (100).

2. Dispositif de protection adaptable (1) selon la revendication 1, comprenant un canal d'échange thermique (50), à l'intérieur de l'enveloppe principale (20) et capable de réguler la température de la partie du corps à protéger (100).

3. Dispositif de protection adaptable (1) selon une ou plusieurs des revendications ci-dessus, dans lequel l'enveloppe intérieure (30) comprend plusieurs bacs intermédiaires (33).

4. Dispositif de protection adaptable (1) selon la revendication ci-dessus, dans lequel ces bacs intermédiaires (33) sont séparés les uns des autres par des parois (32).

5. Dispositif de protection adaptable (1) selon une ou plusieurs des revendications ci-dessus, dans lequel l'enveloppe intérieure (30) est élastique.

6. Dispositif de protection adaptable (1) selon une ou plusieurs des revendications précédentes, dans lequel la première soupape (24) comporte des moyens de connexion avec l'enveloppe de fermeture (40) pour permettre le passage de la première soupape (24) à travers l'enveloppe de fermeture (40) et sa commande depuis l'extérieur.

7. Processus de protection d'une partie du corps à protéger (100) consistant en un dispositif qui ne fait pas partie du corps humain et/ou animal, réalisé au moyen d'un dispositif de protection adaptable (1)
le dispositif de protection adaptable (1) comprend :
- une enveloppe principale (20) imperméable au passage de fluide, souple et définissant un volume principal (21),
- une enveloppe intérieure (30) située à l'intérieur de l'enveloppe principale (20), perméable au passage de fluide et souple ;
- plusieurs particules de remplissage (31) établies dans l'enveloppe intérieure (30) ;
ce processus qui inclut :
- disposer l'enveloppe principale (20), comprenant l'envélo pe interne (30), autour de la partie du corps à protéger (100), définissant une surface interne (22) sensiblement en contact avec la partie du corps à protéger (100) et une surface externe (23) non en contact avec la partie du corps à protéger (100),
- disposer une enveloppe de fermeture (40), imperméable au passage de fluide et souple, autour de la surface externe (23) de l'enveloppe principale (20),
- prévoir des moyens d'étanchéité (41) pour obtenir une étanchéité aux fluides entre l'enveloppe de fermeture (40) et la partie du corps à protéger (41), créant ainsi un volume de fermeture (42) imperméable au passage du fluide et comprenant l'enveloppe principale (20) et la partie du corps à protéger (100),
- dépressuriser le volume de fermeture (42) en définissant une configuration de fermeture dans laquelle l'enveloppe de fermeture (40) est dépressurisée et la pression atmosphérique presse, dans chaque direction, l'enveloppe intérieure (41) contre la partie du corps à protéger (100),
- dépressuriser le volume principal (21), pas avant de dépressuriser le volume de fermeture (42), définissant une configuration comprimée dans laquelle les particules de remplissage (31) sont compactées et définissent sensiblement au moins un corps solide.

8. Processus de protection d'une partie du corps à protéger (100) selon la revendication précédente, comprenant une phase finale de retrait de l'enveloppe (40) de la partie restante du dispositif de protection adaptable (1).
